# EUROPEAN PATENT APPLICATION

(11) **EP 3 566 592 A1**
(43) Date of publication of application: **13.11.2019**
(21) Application number: 18178829.0
(22) Date of filing: 20.06.2018
(51) Int. Cl.: A24D 3/04, A24D 3/06, A24F 47/00

(54) **DUAL-USE CIGARETTE**

(30) Priority: 12.05.2018 CN 201820708779 U
(71) Applicant: Shenzhen Dakavape Tech Co., Ltd., 518000 Shenzhen, Guangdong (CN)
(72) Inventor: WANG, Huayou, Shenzhen, Guangdong 518000 (CN)
(74) Representative: Sun, Yiming

(57) **Abstract**

The present invention discloses a dual-use cigarette, comprising a suckable material segment and a filter tip segment which are wrapped by roll paper, wherein a hollowed segment surrounded by roll paper is disposed between the suckable material segment and the filter tip segment, and the roll paper of the hollowed segment is provided with a plurality of first capillary pores. The dual-use cigarette has the advantages that cold air cannot be sucked when smoking is started, and the burn of a respiratory mucosa caused by high temperature smoke can be avoided.

## Description

### TECHNICAL FIELD

The present invention relates to the field of cigarettes, and in particular to a dual-use cigarette with the advantages that cold air cannot be sucked when smoking is started, and the burn of a respiratory mucosa caused by high temperature smoke can be avoided.

### RELATED ART

With the rising of anti-smoking campaigns at home and abroad, as well as the gradually expanded scope of smoking bans in public places at home and abroad, the research and development of heat not burning (HNB) tobacco products have become one of the research hotspots of tobacco companies in many countries in the world. The so-called HNB tobacco products refer to the tobacco products produced by "heating tobacco shreds or tobacco extract to generate smoke", and avoid high-temperature pyrolysis of tobacco combustion, thereby greatly reducing the amount of release of harmful components in mainstream smoke. Meanwhile, because the tobacco shreds are not burned, the environmental smoke is greatly reduced. However, for the existing dual-use cigarettes, when the smoking is started, cold air will be sucked to cause discomfort. When in heavy smoking, the high-temperature smoke will burn a respiratory mucosa sometimes and cause harm to a human body.

### SUMMARY

An objective of the present invention is to provide a dual-use cigarette with the advantages that cold air cannot be sucked when smoking is started, and the burn of a respiratory mucosa caused by high temperature smoke can be avoided.

The present invention is implemented by the following technical measures: a dual-use cigarette comprises a suckable material segment and a filter tip segment which are wrapped by roll paper, wherein a hollowed segment surrounded by the roll paper is disposed between the suckable material segment and the filter tip segment, and the roll paper of the hollowed segment is provided with a plurality of first capillary pores.

As a preferred embodiment, the roll paper of the filter tip segment is provided with a plurality of second capillary pores.

As a preferred embodiment, a diameter of the first capillary pores is smaller than or equal to a diameter of the second capillary pores.

As a preferred embodiment, the first capillary pores are away from the end of the filter tip segment by 2mm-3mm, a degree of aeration of the first capillary pores is 100CU-600CU, the first capillary pores are in two rows, and a length of the hollowed segment is 5mm-35mm.

As a preferred embodiment, the suckable material segment is a tobacco product segment.

As a preferred embodiment, a suckable material in the suckable material segment is filiform, granular or flaky.

As a preferred embodiment, the roll paper is roll paper for cigarettes.

As a preferred embodiment, the roll paper is roll paper for cigars.

As a preferred embodiment, a length of the filter tip segment is 5mm-25mm, and a suction resistance of the filter tip segment is 2000Pa-5000Pa.

The present invention mainly uses an electric heating device to bake the suckable material segment, such that volatile substances (such as aroma substances and nicotine, etc., of the tobacco) are volatilized for people to suck. Since the hollowed segment is disposed between the suckable material segment and the filter tip segment, the volatile substances (smoke) volatilized by heating before the suction are stored in the hollowed segment, and the scented gas can be sucked when people start to suck. Since the plurality of first capillary pores is disposed in the roll paper of the hollowed segment, the cold air in the hollowed segment originally is discharged from the first capillary holes while the volatile substances are stored in the hollowed segment, so as to avoid the discomfort caused by suction of the cold air when the smoking is started. Due to the small aperture of the small first capillary pores, the gas flow can be formed only under a certain pressure difference. The plurality of first capillary pores is disposed in the roll paper of the hollowed segment, and during the suction, the cold air from the outside can be sucked to cool the sucked high-temperature smoke, and the high temperature smoke is prevented from burning the respiratory mucosa.

### BRIEF DESCRIPTION OF THE DRAWINGS

Fig. 1 is a sectional view of an embodiment of the present invention.
Fig. 2 is an enlarged view of a part A of Fig. 1.

### DETAILED DESCRIPTION

The present invention is further explained in detail in combination with the embodiments and in accordance with the accompanying drawings.

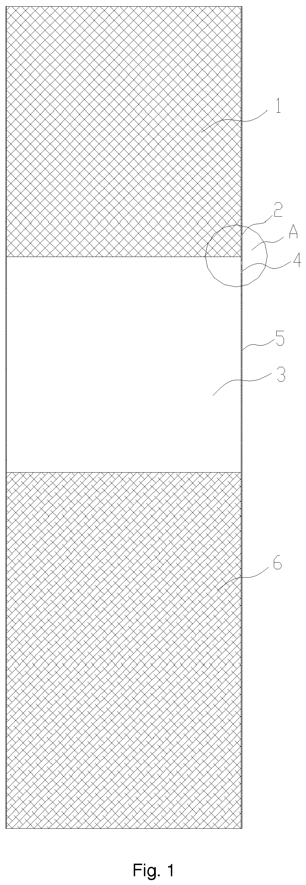

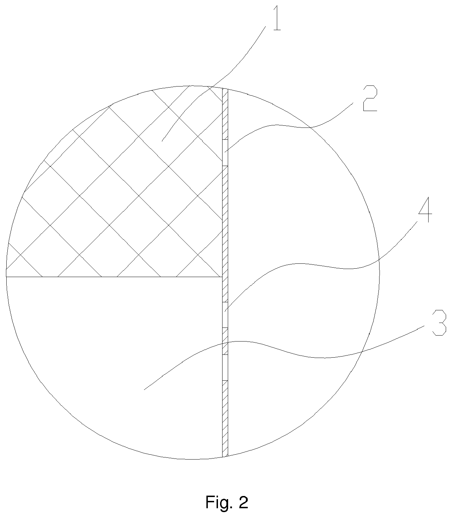

A dual-use cigarette, referring to Figs. 1 and 2, comprises a suckable material segment 6 and a filter tip segment 1 which are wrapped by roll paper 5, wherein a hollowed segment surrounded by the roll paper 5 is disposed between the suckable material segment 6 and the filter tip segment 1, and the roll paper of the hollowed segment is provided with a plurality of first capillary pores 4.

The present invention mainly uses an electric heating device to bake the suckable material segment, such that volatile substances (such as aroma substances and nicotine, etc., of the tobacco) are volatilized for people to suck. Since the hollowed segment is disposed between the suckable material segment and the filter tip segment, the volatile substances (smoke) volatilized by heating before the suction are stored in the hollowed segment, and the scented gas can be sucked when people start to suck. Since the plurality of first capillary pores is disposed in the roll paper of the hollowed segment, the cold air in the hollowed segment originally is discharged from the first capillary holes while the volatile substances are stored in the hollowed segment, so as to avoid the discomfort caused by suction of the cold air when the smoking is started.

Due to the small aperture of the small first capillary pores, the gas flow will be formed only under a certain pressure difference. The plurality of first capillary pores is disposed in the roll paper of the hollowed segment, and during the suction, the cold air from the outside can be sucked to cool the sucked high-temperature smoke, and the high temperature smoke is prevented from burning the respiratory mucosa.

According to the dual-use cigarette of the present invention, referring to Fig. 1 to Fig. 2, based on the foregoing technical solution, specifically, the roll paper 5 of the filter tip section 1 is also provided with a plurality of second capillary pores 2. By using the second capillary pores 2 in the roll paper 5 of the filter tip section 1, the smoke is further cooled, and the comfort level of the smoke entering an oral cavity is ensured. In order to guarantee the full gas suction of the second capillary pores 2 of the filter tip section 1, a diameter of the first capillary pores 4 is set to be smaller than or equal to a diameter of the second capillary pores 2.

According to the dual-use cigarette of the present invention, referring to Fig. 1 to Fig. 2, based on the foregoing technical solution, specifically, the first capillary pores 4 are away from the end of the filter tip segment 1 by 2mm-3mm, a degree of aeration of the first capillary pores 4 is 100CU-600CU, the first capillary 4 pores are in two rows, and a length of the hollowed segment is 5mm-35mm.

According to the dual-use cigarette of the present invention, referring to Fig. 1 to Fig. 2, based on the foregoing technical solution, specifically, the suckable material segment 1 is a tobacco product segment.

According to the dual-use cigarette of the present invention, referring to Fig. 1 to Fig. 2, based on the foregoing technical solution, specifically, a suckable material in the suckable material segment 1 is filiform, and may also be granular or flaky in other embodiments.

According to the dual-use cigarette of the present invention, referring to Fig. 1 to Fig. 2, based on the foregoing technical solution, specifically, the roll paper 5 is roll paper for the cigarettes. In order to improve quality of the smoke, in other embodiments, the roll paper may also be roll paper for cigars.

According to the dual-use cigarette of the present invention, referring to Fig. 1 to Fig. 2, based on the foregoing technical solution, specifically, a length of the filter tip segment is 5mm-25mm, and a suction resistance of the filter tip segment is 2000Pa-5000Pa.

The foregoing elaborates the two-dual cigarette according to the present invention and is intended to help to understand the present invention. However, the embodiments of the present invention are not limited to the above embodiments, and any changes, modifications, substitutions, combinations and simplifications made without departing from the principle of the present invention should be equivalent replacement modes and are included within a protective scope of the present invention.

## Claims

1. A dual-use cigarette, **characterized by** comprising a suckable material segment and a filter tip segment which are wrapped by roll paper, wherein a hollowed segment surrounded by the roll paper is disposed between the suckable material segment and the filter tip segment, and the roll paper of the hollowed segment is provided with a plurality of first capillary pores.

2. The dual-use cigarette according to claim 1, **characterized in that** the roll paper of the filter tip segment is provided with a plurality of second capillary pores.

3. The dual-use cigarette according to claim 2, **characterized in that** a diameter of the first capillary pores is smaller than or equal to a diameter of the second capillary pores.

4. The dual-use cigarette according to claim 1, **characterized in that** the first capillary pores are away from the end of the filter tip segment by 2mm-3mm, a degree of aeration of the first capillary pores is 100CU-600CU, the first capillary pores are in two rows, and a length of the hollowed segment is 5mm-35mm.

5. The dual-use cigarette according to claim 1 or 2, **characterized in that** the suckable material segment is a tobacco product segment.

6. The dual-use cigarette according to claim 1 or 2, **characterized in that** a suckable material in the suckable material segment is filiform, granular or flaky.

7. The dual-use cigarette according to claim 1 or 2, **characterized in that** the roll paper is roll paper for cigarettes.

8. The dual-use cigarette according to claim 1 or 2, **characterized in that** the roll paper is roll paper for cigars.

9. The dual-use cigarette according to claim 1 or 2, **characterized in that** a length of the filter tip segment is 5mm-25mm, and a suction resistance of the filter tip segment is 2000Pa-5000Pa.
